Europäisches Patentamt

European Patent Office

Office européen des brevets

(11) Publication number: **0 227 180**
**A2**

# EUROPEAN PATENT APPLICATION

(21) Application number: **86202275.3**

(22) Date of filing: **16.12.86**

(51) Int. Cl.⁴: **G10K 11/08 , A61F 11/00**

(30) Priority: **17.12.85 US 810039**

(43) Date of publication of application:
**01.07.87 Bulletin 87/27**

(84) Designated Contracting States:
**AT BE CH DE ES FR GB GR IT LI LU NL SE**

(71) Applicant: **SOUND ACOUSTIC SYSTEMS, LTD.**
**24422 Avenida de la Carlotta**
**Laguna Hills California(US)**

(72) Inventor: **Colluci, Dennis A.**
**24802 La Paz**
**Dana Point California 92526(US)**
Inventor: **Batista, Roland E.**
**323 Elati Court**
**Danville California 94526(US)**

(74) Representative: **de Boer, Hindrik Geert Jan et al**
**de Boer & Wigman P.O. Box 5265 De Lairessestraat 131-135**
**NL-1007 AG Amsterdam(NL)**

(54) Improved hearing aid device.

(57) A non-electric hearing aid device formed to fit within the human ear, amplifies and redirects acoustic waveforms by use of a thin-walled resonating element and a frustro-conical shaped metal directional element. The thin-walled resonating element is formed of a stiff, smooth plastic material and has an interior cavity including two openings to acoustically communicate therethrough. The metal directional element is contiguous with and depends inwardly from a forward facing opening into the cavity.

Fig.1

EP 0 227 180 A2

## IMPROVED HEARING AID DEVICE

This invention relates generally to hearing aid devices and, more particularly, to a non-electrical acoustic resonating system for insertion into the human ear to provide natural audio amplification for users with mild hearing loss.

In the field of acoustic amplification systems to improve human hearing there are generally two types of prior art systems, namely electrical and non-electrical systems. Because of the recent advances in the miniaturization of electrical components, the present state hearing amplification systems have greatly improved and have been directed primarily to electrical prior art systems. However, there are deficiencies in such electrical prior art systems. Generally speaking, the electronic or electrical prior art hearing aid or acoustic aid systems are generally very costly. Furthermore, such systems generally require an examination, prescription, and installation by a highly trained technician. Finally, the electrical or electronic type amplification system is not always appropriate for mild hearing loss since the electrical systems generate their own electronic noise which users may find uncomfortable and/or distracting. When this noise is filtered, low intensity acoustic signals may also be reduced by the filtering system or by the resultant failure of the hearing aid to simulate the signals because of the hearing aid's signal to noise ratio. Further, such prior art devices often possess excessive acoustic power, rendering the same inappropriate for many hearing loss applications.

In addition to the electrical or electronic amplification systems, the prior art is filled with numerous non-electrical amplification systems. These take various forms, but more commonly they are merely megaphone type systems which have one opening to be inserted into the external ear canal and the other open end to be directed at the sound source which is desired to be heard. However, such large devices are generally unsightly, aesthetically unpleasing and rarely recommended for use.

Therefore, there exists a substantial need in the art for a non-electric hearing aid device which can be used in mild hearing loss situations to provide acoustic gain at a reduced cost and which can be effectively dispensed. Specifically, there is a need for a means for acoustic amplifications to primarily bolster the mid-frequency peak gain within the normal speech range above 850Hz while reducing late reverberations.

The present invention specifically overcomes the difficulties of the previous hearing aid systems by being able to non-electronically aid hearing specifically within the normal speech ranges. Thus, unlike conventional prior art systems, the present invention provides an aesthetic, easily insertable, non-electronic hearing aid device which is primarily designed to boost mid-frequency acoustic signals thereby increasing hearing perception for important frequencies inherent in the normal speech range.

To achieve the increased peak levels in the normal speech ranges, the present invention comprises a resonator shell element in acoustic communication with the external ear canal of the human subject. The resonator shell element is formed in a shape and uses appropriate materials to optimize the amplification by incorporating features which maximize amplification while minimizing acoustic detracting factors. Thus, the present invention is able to achieve the desired effects of increasing peak level within the frequencies normally attributable to normal speech ranges.

Furthermore, the present invention incorporates a metal directional element having a specific shape, material, and disposition within the resonator shell to redirect normal acoustical incoming signals. Additionally, when the device is inserted into the ear a reduction in higher frequency late reverberation is obtained. As a result, the present invention provides a simple, easy to administer, inexpensive and readily available non-electronic hearing aid device to fill the needs as earlier described.

These, as well as other features of the present invention will become more apparent upon reference to the drawings, wherein:

Figure 1 is a perspective side view of the present invention as inserted in the human ear;

Figure 2 is a front perspective view of the resonator shell element;

Figure 3 is a rear perspective view of the resonator shell element;

Figure 4 is a cross-sectional view taken along lines 4-4 of Figure 2;

Figure 5 is a graph illustrating the relationship between susceptance conductance and admittance;

Figure 6 is a graph illustrating the interrelationships of mass and stiffness upon the reactance as a function of frequency; and

Figure 7 is a graph illustrating the acoustical pattern of the present invention.

The present invention comprises a non-electronic acoustic resonating device which fits discretely in the ear of a user to provide natural audio amplification without distortion for users having mild hearing loss. More particularly, the present invention increases the effective volume of the ear canal of a user to produce a downward shift or change in the resonance frequency of the ear which aids in improving hearing perception.

By way of background, the external ear, head and upper torso of a user provide directional amplification of audio signals dependent upon the frequency composition of the audio signal. The auditory canal of the ear further modifies the audio spectrum through its natural resonance properties. The resonance of the concha further provides an acoustic gain at the entrance of the auditory canal, relative to the sound field in the frequency range of 2.5-4 KH₂.

Insertion of the present invention into the ear of a user results in an auditory coupling of the two resonating cavities, i.e. the resonating cavity of the present invention with the natural resonating cavity of the ear of a user. This coupling thereby increases the effective volume of the resonating cavity of the ear and thereby produces spectral emphasis in the desired mid-frequency range of hearing typically associated with normal speech. This spectral emphasis is defined by the well known Helmholtz resonation formula, namely:

$$f \;=\; \frac{v}{2\pi}\sqrt{\frac{a}{VL}}$$

where f is the resonance frequency of the system; $v$ is the speed of sound; V is the volume of the cavity; L is the length of the entrance neck and a is the area of the entrance of the neck.

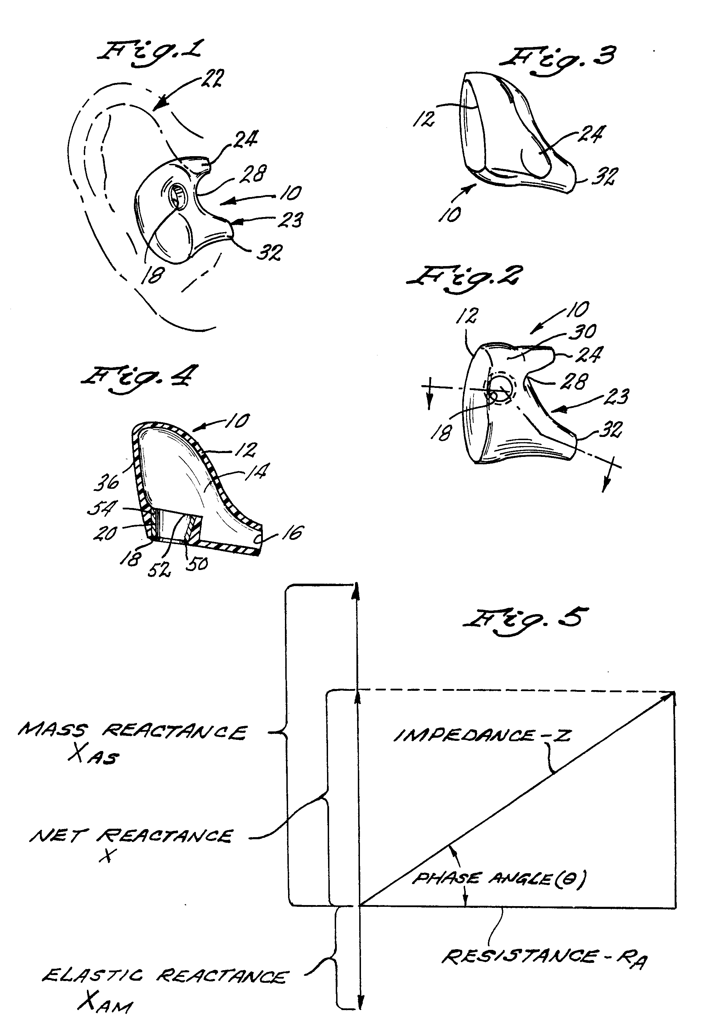

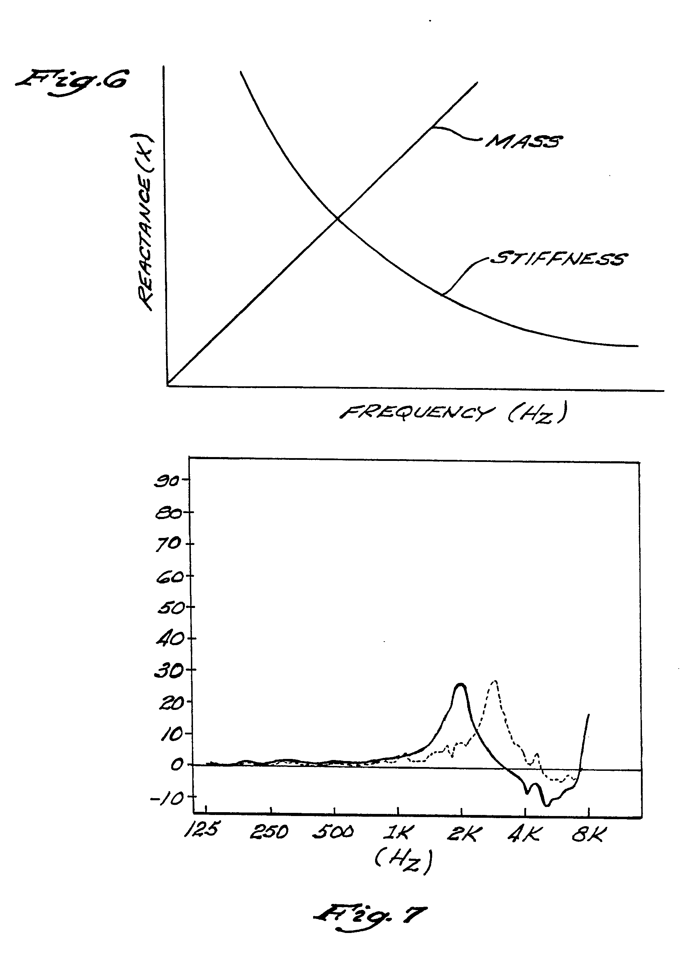

Referring to Figures 1-4, there is shown the improved hearing device 10, of the present invention, comprising: a resonator shell 12, formed to define an interior cavity 14, having a first opening 16, and a second opening 18; and a metal directional element 20 disposed therein. In order to achieve the desired amplification objectives of the present invention and to transverse the deficiencies as perceived in the prior art, the present invention considered not only the physical parameters of the elements in regard to directionality and orientation, but also took into consideration the materials used in constructing such elements in order to maximize the amplification qualities and to minimize acoustic losses.

Referring to Figures 1 and 2, a more detailed description of the external topography of the resonator shell 12 of the present invention is presented. The external topography of the resonator shell 12 is formed to be received or fitted into a respective outer ear (pinna) 22 of a user. More specifically, the shell 12 includes a resonating surface 23 which interfaces or engages with the human subject's ear and possesses a saddle-shaped contour having a first apex or registry tab 24 formed to fit into or engage the anatomical structure known as the cymba 26 of the ear, sloping to an integral depression 28, shaped to fit over the crus helias 30 of the ear and rising to a second apex or registry tab 32 formed to be inserted into the external ear canal - (not shown) of the ear. By this particular shape, the shell 12 can removably engage the pinna 22 and thus be maintained in a desired orientation within the ear. This prevents any inadvertent shifting and thus, loss of directionality is minimized.

Disposed at the second apex or registry tab 32, is the first opening 16 having an inside diameter of approximately 4.0 ± 1.0 millimeters, which provides a means for acoustic communication between the external ear canal and the interior cavity 14 formed within the resonator shell element 12. Although preferably the opening is formed in a circular configuration, the opening 16 may be formed in alternative irregular configurations showing effective cross-sectional area dimensions. In the preferred embodiment, the shell 12 comprises a thin-walled plastic element typically comprising a flesh-tone acrylic plastic material having a wall thickness of approximately 0.015 ± .010 inches. As shown more clearly in Figure 4, the cavity 14 is generally frustro-conical in shape, with the opening 16 being disposed adjacent the apex of the conical frustro-structure and the wall 36 opposite the opening 16 forming the base of the frustro-conical shaped section. The frustro-conical shape of the cavity 14 serves to redirect and amplify incoming waveforms from the forward facing opening 18 which is typically positioned approximately 90° (i.e. normal) to the ear canal. In the preferred embodiment, the volume of the cavity 14 is between 1.4 and 3.0 cubic centimeters. By varying the volume, as described later, the acoustic characteristics of the hearing device 10 may be altered. Disposed substantially perpendicular to the first opening 16, in a forward facing direction in the wall 30, is the second opening 18. The second opening 18 is positioned in the upper one-third portion of the wall 30 to avoid being eclipsed by the anatomical ear feature known as the tragus 40 to maintain a low profile relative to the ear and yet face unobstructed in an essentially forward direction. By this construction, an unobtrusive forward directionality is achieved to facilitate the amplification properties of the present invention.

The metal directional element 20 depends inwardly from and contiguous with the second opening 18 and comprises of a frustro-conical tubular element having a first opening 50, having an inside diameter of approximately 0.150 ± .050 inches and a second, larger opening 52 having an inside diameter of 0.175 ± .050 inches. The openings 50 and 52 are axially separated from one another by a distance of 0.105 ± .050 inches by a tapered transitional surface 54 of approximately five (5) degrees. By this construction, the metal directional element 20 provides a means for directionally communicating acoustic waveforms from the environment into the cavity 14.

The aforementioned disposition of the various elements of the present invention allows the device of the present invention 10 to be positioned within the ear so that the device 10 can unobtrusively reside within the ear and yet still be directed in a forward direction to redirect and amplify the incoming acoustic signal. By using a combination of two frustro-conical shapes, the present invention concurrently increases the peak gain while redirecting the sound waves 90° into the external ear canal.

Furthermore, in order to view the desired amplifications and frequency objections as earlier described, the physical materials used to make or construct the device are selected to magnify the beneficial acoustic effects and to minimize the acoustic effect detractors. Thus, in selecting the particular materials for use in the constructions of the present invention, the resistance and reactance of the particular elements are taken into consideration.

Referring to Figure 5, a more detailed description of the interrelationships of friction, inertia, and stiffness is present. Impedance is the interaction of resistance $R_A$, positive reactance $X_{AS}$ and negative reactance $X_{AM}$. These components are related to friction, mass, and stiffness. Frictional resistance or attenuation of the sound waves as they: (1) impinge upon the present invention's surfaces; (2) are distorted by their passage through openings 16 and 18; and (3) are perpendicularly redirected from a forward facing direction to the external ear canal. Friction causes a portion of the energy applied to the system to be converted into heat. This dissipation of energy into heat is termed resistance. Resistance is not related to frequency, and occurs in phase with applied force. It is shown on the x-axis in Figure 5.

Reactance is the storage, as opposed to the dissipation, of energy by the system. Mass - (positive reactance or negative susceptance) is associated with the mass of the system. Since all mass has the property of inertia, the application of a force F to the mass M causes the mass to accelerate according to the formula F = MA - (where A is acceleration). If the force is applied sinusoidally, the mass reactance ($X_{AM}$) is related to frequency as $X_{AM} = 2 \pi fM$, where f is frequency. Thus, as depicted in Figure 6, the magnitude of $X_{AM}$ is directly proportional to frequency (the higher the frequency, the greater the mass reactance).

Stiffness $X_{AS}$ (negative reactance or positive susceptance) is the reluctance of the material to deform and transform the acoustic wave form into a mechanical form. If one views stiffness or negative reactance as the reluctance of a spring to compress, applying a force F, compresses (displaces) the spring according to the formula F = SD, where D is the amount of displacement and S is the elasticity of the system. When the stimulus is applied sinusoidally, $X_{AS}$ is related to frequency as $X_{AS} = s/(2 \pi f)$. In other words, as depicted in Figure 6, the amount of stiffness (negative reactance or positive susceptance) is inversely proportional to frequency ($X_{AS}$ decreases as frequency increases). Since $X_{AM}$ is proportional to frequency, they should be equivalent at some frequency. This is the system's resonant frequency, at which point the reactance or susceptance components cancel each other out, leaving only the resistance component. Thus, by altering the reactance components - (stiffness and mass) and reducing the resistive component (friction) the resonance frequency and the gain can be altered as desired.

Since the elasticity and mass components are 180° out of phase due to displacement leading and lagging behind the force applied, a system's net reactance is equal to the difference between them. This relation is shown in Figure 5 for the condition where $X_{AS}$ exceeds $X_{AM}$ which is the case for the present invention. Notice that the overall impedance results from the interaction between the resistance and the net reactance. As in any other system, the impedance of the present invention is due to its stiffness (elasticity), mass (inertia) and resistance (friction).

In the light of these principles, the resonator element 12 is formed of an acrylic plastic material having extremely low-mass which is concurrently structurally stiff. Thus, the selective use of the constructive materials minimizes both the reactance and resistance of the particular wave passing through the resonator element. Likewise, the metal directional element 20 is formed of a light, stiff metal which in the preferred embodiment is aluminum, although any light, stiff metal can be used. As such, the specific structural parameters as well as the materials selected to construct the present invention were selected to minimize losses due to inertia (mass), elasticity (stiffness) and friction. The resulting device thus non-electronically provides acoustic gain.

In operating, the hearing aid device 10 of the present invention is unobstrusively inserted into the appropriate ear with the opening 18 being disposed in a forward facing direction. By such insertion, incoming acoustical sound waves are unobstructively directed toward the forward facing opening 18 by turning of the head of the human subject. As the particular acoustic sound waves reach the opening 18, they enter through the metal directional element 20 and into the resonator shell 14 and are reflected and directed by the resonator shell element 12 down and into the external ear canal of the human subject. Due to the principles defined by the Helmholtz equation, as the incoming acoustic signal enters into the interior of the resonating shell 12, the volume of the ear canal is coupled to the volume of the interior of the resonating shell 12, which produces a downward shift in the resonance frequency of the ear. The downward shift thereby changes the hearing perception of the mild hearing loss user.

Referring more specifically to Figure 7, the acoustical resonance frequency shift made possible by the present invention is depicted. The solid line represents one embodiment of the present invention having a cavity 14 of 2.6 cubic centimeters. A 30 decibel peak is depicted at about 2000Hz. The phantom line of Figure 7 depicts the analogous sound pattern received after decreasing the cavity 14 volume of the present invention from 2.6 cubic centimeters to 1.4 cubic centimeters. Thus, it can be observed that a particular downward frequency shift of approximately 1200Hz results by increasing the volume of the resonance system by 1.2 cubic centimeters.

As a cumulative result of the sound reflection, resonance and frequency shift of the present invention compounded by the variation of the reactive and resistive elements from that of the concha to that of the present invention, an increased peak gain occurs between 850-2500 hertz (Hz). More specifically, a peak gain between 12 and 17 decibels between 1400 and 2000 Hz for an average sized ear is realized. Additional peak resonance above 2000 Hz is present in response to normal head movement, varying the peak gain from 0-18 dB as the directionality of the receiver is changed.

The presence of additional mid-frequency gain provided by the present invention improves the signal-to-noise ratio for listening situations. Additionally, voice monitoring can be enhanced due to a greater identification of mid-frequency speech sounds by the user. Furthermore, the variable high frequency response provided by the present invention can help to improve sound localization, which can be helpful in the understanding of words in background noise.

In conclusion, in the present invention, a resonator shell element and a metal directional element are carefully designed to provide a desired spectral shift in peak gain without the use of electronic amplification. The performance is enhanced by the minimizing of acoustic wave detractors of mass, elasticity and friction by the selection of the materials and in the actual construction of the particular device. As a result, the present invention amplifies the sound waves associated within the speech range so as to provide an inexpensive means to effect a change in hearing perception.

Having described a preferred embodiment of the present invention, various modifications will now become apparent from this specification and the drawings which fall within the scope of the amended claims.

## Claims

1. A non-electronic hearing aid device comprising:
cavity means formed to be inserted within the ear of a user of artificially decreasing the resonance frequency of the ear; and
means disposed within said cavity means for directing acoustic signals within the interior of said cavity means.

2. The device of Claim 1 wherein said cavity means comprises a resonator shell element, formed to define a cavity having a pair of openings to acoustically communicate through said cavity, one of said openings disposed in a substantially forward facing direction and the other of said openings extending into the ear canal when said cavity means is inserted within the ear of a user.

3. The device of Claim 2 wherein said directing means comprises a metal directional element contiguous with and depending from said one of said openings.

4. The device of Claim 3 wherein the metal directional element comprises a frustro-conical shaped tubular element having a length of 0.150 ± .050 inches and a diameter of 0.105 ± .050 inches and 0.175 ± .050 inches at opposite ends.

5. The device of Claim 4 wherein the metal directional element is formed of a light, rigid and polished material.

6. The device of Claim 5 wherein the metal directional element is formed of aluminum.

7. The device of Claim 6 wherein the resonator shell element comprises a thin walled plastic element.

8. The device of Claim 7 wherein said cavity comprises a frustro-conical shaped cavity having a volume of approximately 1.4 to 3.0 cubic centimeters.

Fig. 1

22
24
28
10
23
32
18

Fig. 3

12
24
32
10

Fig. 2

12
10
30
24
28
23
32
18

Fig. 4

10
12
14
16
36
54
20
18  52  50

Fig. 5

MASS REACTANCE
$X_{AS}$

NET REACTANCE
$X$

ELASTIC REACTANCE
$X_{AM}$

IMPEDANCE - Z

PHASE ANGLE (θ)

RESISTANCE - $R_A$

*Fig.6*

FREQUENCY (Hz)

*Fig. 7*